# EUROPEAN PATENT SPECIFICATION

(11) **EP 1 559 421 B1**
(45) Date of publication and mention of the grant of the patent: **27.07.2016**
(21) Application number: 03810580.5
(22) Date of filing: 03.09.2003
(51) Int. Cl.: A23L 2/52, A61K 31/192, A61K 31/216, A61P 7/00, A61P 9/10

(54) **BLOOD CIRCULATION PROMOTING AGENT**
MITTEL ZUR FÖRDERUNG DER BLUTZIRKULATION
AGENT PROMOTEUR DE LA CIRCULATION SANGUINE

(30) Priority: 06.11.2002 JP 2002322474; 07.11.2002 JP 2002323623
(43) Date of publication of application: 03.08.2005
(73) Proprietor: KAO CORPORATION, Tokyo 103-8210 (JP)
(72) Inventor: ARAI, Youichi, Tokyo 131-8501 (JP); WATANABE, Takuya, Tokyo 131-8501 (JP); SUZUKI, Atsushi, HAGA-GUN, Tochigi 321-3497 (JP); JOUKURA, Hiroko, HAGA-GUN, Tochigi 321-3497 (JP)
(74) Representative: Hoffmann Eitle
(86) International application number: PCT/JP2003/011244
(87) International publication number: WO 2004/041265

(56) References cited:
- WO-A2-02/42429
- JP-A- 4 243 822
- JP-A- 2002 145 767
- JP-A- 2002 371 002
- US-B1- 6 447 814
- HSIEH, MING-TSUEN ET AL: "Effects of ferulic acid on the impairment of inhibitory avoidance performance in rats" PLANTA MEDICA , 68(8), 754-756 CODEN: PLMEAA; ISSN: 0032-0943, 2002, XP002584780
- DATABASE CA [Online] CHEMICAL ABSTRACTS SERVICE, COLUMBUS, OHIO, US; 2001, LI, SHANGZHU ET AL: "Effect of sodium ferulate on the circulating endothelial cells from patients with progressive systemic sclerosis" XP002584781 retrieved from STN Database accession no. 2001:218275 & ZHONGHUA PIFUKE ZAZHI , 34(1), 34-35 CODEN: CHFTAJ; ISSN: 0412-4030, 2001,
- KOJIMA, S. ET AL: "Effects of Shimotsu-to on the microcirculation of the bulbar conjunctiva and hemorheological parameters in normal subjects" PHYTOMEDICINE , 5(1), 19-24 CODEN: PYTOEY; ISSN: 0944-7113, 1998, XP009134113
- DATABASE CA [Online] CHEMICAL ABSTRACTS SERVICE, COLUMBUS, OHIO, US; 1987, XU, JINGFENG ET AL: "Effect of ethanolamine nitrate ester salt of ferulate on the cardiovascular system" XP002584783 retrieved from STN Database accession no. 1988:16033 & YIYAO GONGYE , 18(9), 410-13 CODEN: YIGODN; ISSN: 0255-7223, 1987,
- DATABASE WPI Section Ch, Week 199001 21 November 1989 (1989-11-21), Thomson Scientific, London, GB; Class D13, AN 1990-005149 XP002584784 & JP 01 289449 A (RATSUKU KESHOHIN KK) 21 November 1989 (1989-11-21)

## Description

### Field of the Invention

The present invention relates to a non-therapeutic use of a chlorogenic acid for promoting the blood circulation.

### Background of the Invention

The circulation of blood is greatly involved in the roles of supplying human body tissues with oxygen and nutrients while excreting waste products therefrom, thereby performing important functions therein.

Recently, in our life environments, increasingly more people suffer from poor circulation at various parts of their bodies as they get involved increasingly in circumstances where they remain in a fixed posture for extended periods of time, owing to introduction of computers and other information equipment or due to other reasons. A seasonal change also brings about changes in our vital life, for example, it may often invites poor circulation in peripheral circulatory system in a winter season. Further no one can avoid deterioration of physical functioning owing to his aging, and thus poor circulation due to aging is a great concern as well.

Such poor circulation caused by life environments, seasonal changes, aging and other various factors may threaten the vital activity at various body parts and in many cases result in disorders of mind and body.

Ingredients known heretofore as effective in promoting the blood circulation or improving the blood fluidity which is a factor of blood circulation promotion include: collagen peptide (TP-A-2002-121148); Bidens plants, especially Bidens pilosa or its ingredients (JP-A-2002-205954); γ-linolenic acid as used singly or in combination with a fat-soluble antioxidant (JP-A-2000-302677); dilazep and its acid addition salts (JP-A-1999-92382); hydroxymehylfurfural derivatives (JP-A-1999-228561); estrogen agonists (JP-A-1998-7564); fermented vinegars (JP-A-1998-28567); mulberry leaves, Japanese apricot kernels, Japanese apricot pulps, beefsteak plant leaves, and like materials (JP-A-1998-127253); plasmins and plasminogen activators (JP-A-1996-40931); hyaluronic acids (JP-A-1996-53356); and bilobalides contained in ginkgo leaves (JP-A-1995-53371).

The cerebral apoplexy is a kind of cerebrovascular diseases and is generally classified into cerebral infarction, cerebral hemorrhage and so forth. Statistics reveals that in Japan the number of fatalities due to cerebral apoplexy is so large that this disease ranks third following cancers and ischemic heart diseases that are ranked first and second, respectively.

Risk factors of cerebrovascular diseases may include hypertension, diabetes, hyperlipidemia, smoking, alcohol drinking, obesities and stresses which are factors typically resulting from our daily lifestyle habits and it is apprehended that those risk factors will grow and increase as the society becomes aged.

Conventionally, active ingredients for the prevention of cerebrovascular diseases are available, for example, in the form of calcium antagonists, ACE inhibitors, αβ blockers, etc. that are used as drug medicines, while for this purpose it is also proposed to use glycerophoholipids having fatty acid residues containing a docosahexaenoyl group(s) (JP-A-2000-239168), MCP-1 (Monocyte Chemotactic Protein-1) inhibitors (JP-A-1999-60502), compounds having an anti-endothelin action (JP-A-1998-72363), chitosan (JP-A-1998-182469), activated protein C (JP-A-1995-233087), haptoglobins (JP-A-1994-128173), etc.

### Disclosure of the Invention

However, as matters stand now, those medicines used for improving the blood fluidity, promoting the blood circulation or improving cerebrovascular diseases are satisfactory as far as their therapeutic efficacies are concerned, but nevertheless patients tend to be heavily burdened with such side effects as hemostatic difficulties, excessive pressure drops during nighttime, low blood pressure, dry coughs, headaches, vertigoes, etc. that are more or less involved in the use of such drug medicines. Meanwhile, those foods or their active ingredients which have been said to be effective in improving the blood fluidity, promoting the blood circulation or improving cerebrovascular diseases are not always satisfactory in respect of their efficacies, and further, it takes a longer time for them to exhibit such effects of improving the blood fluidity, promoting the blood circulation, or improving cerebrovascular diseases.
WO 02/42429 relates to the therapeutic use of chlorogenic acid e.g. for the treatment of Alzheimer's disease, type II diabetes and the like.
JP 19880118250 teaches to include a gold foil into a coffee drink which helps to accelerate the blood circulation.

Accordingly, an object of the present invention is to provide a non-therapeutic use of a chlorogenic acid wherein the acid does not only work highly effectively to improve the blood fluidity, and to promote the blood circulation, but also has an excellent safety and thus hardly place any substantial burdens on users in daily ingestion.

With a view to achieving the aforementioned object, the inventors undertook a series of studies from various aspects to search for certain ingredients which are effective in promoting the blood circulation out of ingredients which can be taken in or ingested over a long period of time and are highly safe to use. As a result, they found out that one or more ingredients selected from the group consisting of chlorogenic acids, and pharmaceutically acceptable salts of these acids can be effectively used as a blood circulation promoter.

The inventors have also found out that administering one or more ingredients selected from chlorogenic acids, and pharmaceutically acceptable salts thereof to persons affected by their lowered functions of peripheral circulation improves their peripheral circulation, so that their cold hands and feet and their body temperature decrease can be inhibited. Further, the inventors have found out that the above-described ingredients according to the present invention are substantially free from such side effects as observed in medicines in general and may be easily taken in daily life and thus can be usefully applied in the field of health foods.

Specifically, the present invention provides the non-therapeutic use of a chlorogenic aicd as defined in the claims for promoting blood circulation.

Since the use of chlorogenic acids improves the blood fluidity, blood circulation or body coldness or inhibits body temperature decrease, these agents are usefully applied for the prevention and treatment of any poor blood circulation caused by life environments, seasonal changes or aging. The above-described acids are highly safe and can be orally taken over long periods of time and thus can have useful applications in functional foods and in foods for specified health use besides medicines.

### Detailed Description of the Invention

The chlorogenic acids, used in the present invention may be extracted from natural products, especially plants, containing such acids or may be industrially produced by chemical synthesis.

The chlorogenic acids are present as stereoisomers and the present invention may employ these acids in the form of either pure isomers or mixtures thereof. Specifically, the chlorogenic acids includes 3-caffeoylquinic acid, 4-caffeoylquinic acid, 5-caffeoylquinic acid, 3,4-dicaffeoylquinic acid, 3,5-dicaffeoylquinic acid, 4,5-dicaffeoylquinic acid, 3-feruloylquinic acid, 4-feruloylquinic acid, 5-feruloylquinic acid, and 3-feruloyl-4-caffeoylquinic acid (cf. "Chemistry and Technology of Coffee Roasting", by Nakabayashi et al., KOGAKU SHUPPAN CO., LTD., p166-167).

When salified, these chlorogenic acids can enhance their water solubility and increased physiological efficacy. As for salts of these acids, any pharmaceutically acceptable salts may be used for the present invention. Basic substances suitably employed to form such salts of the acids described above include, for example, alkali metal hydroxides such as lithium hydroxide, sodium hydroxide, potassium hydroxide, etc.; hydroxides of alkaline earth metals such as magnesium hydroxide, calcium hydroxide, etc.; inorganic alkalis such as ammonium hydroxide, etc.; basic amino acids such as arginine, lysine, hystidine, ornithine, etc.; and organic alkalis such as monoethanolamine, diethanolamine, triethanolamine, etc.; and the hydroxides of alkali metals and alkaline earth metals are particularly preferred. For the present invention, any such salts prepared beforehand may be added to a composition containing the remaining ingredients, or alternatively any chlorogenic acids and salt forming ingredients may be separately added to such a composition so that salts are formed therein.

According to the present invention, preferable natural product extracts, particularly plant extract, containing chlorogenic acids include, for example,: extracts of coffees, cabbages, lettuce, artichokes, tomatoes, eggplants, potatoes, carrots, apples, pears, plums, peaches, apricots, cherries, sunflowers, Jew's marrow, sugarcanes, nandina leaves, blueberries, wheats, etc.

For example, the chlorogenic acids may preferably be extracted from plant matters such as green coffee beans, nandina leaves, unripe apple fruits, etc. More preferably, the chlorogenic acids may be extracted from seeds of Coffee arabica LINNE with a warmed acidic aqueous solution of ascorbic acid or citric acid or with hot water.

More specifically, preferable extracts of green coffee beans include "Flavor Folder" supplied by T. Hasegawa Co., Ltd., Tokyo, Japan and preferable apple extracts include "Applephenon" supplied by THE NIKKA DISTILLING CO., LTD., Tokyo, Japan and preferable sunflower seed extracts include "Heliant" by DAINIPPON INK AND CHEMICALS, INC., Tokyo, Japan.

The above-described ingredients of the present invention may be used in combination of two or more of them. These ingredients are ingested in a total intake ranging preferably from 10 mg to 10 g, more preferably from 35 mg to 5 g and further preferably from 70 mg to 1 g per adult (weighing 60 kg) daily in order to achieve actions to improve the blood fluidity and promote the circulation as intended.

The rheological property of blood is important as a passive factor of blood circulation, especially in microcirculation. For blood capillaries, microarteries and microveins, for example, mechanical properties of blood cells such as erythrocyte deformability and leukocyte adhesivity constitute major factors governing the blood rheology and it is presumed that microcirculation disorders due to any anomalies of such factors constitute causes and symptoms of many diseases.

For measuring effects achieved by the above-described ingredients to improve the blood fluidity, a number of methods may be employed
including: microchannel method (cf. "Optically accessible microchannels formed in a single-crystal silicon substrate for studies of blood rheology" by Kikuchi, Y., Sato, K., Ohki H. and Kaneko T. in Microvasc. Res. 44, 226-240 (1992)); laser diffractometry (cf. "Modulation of erythrocyte membrane mechanical function by beta-spectrin phosphorylation and dephosphorylation" by Manno S., Takakuwa Y., Nagao K., Mohandas N. in J. Biol.Chem.270(10), 5659-5665 (1995)); filter method (cf. "Regulation of red blood cell filterability by Ca2+ influx and cAMP-mediated signaling pathways" by Oonishi T., Sakashita K., Uyesaka N. in Am.J.Physiol.273(6), C1828-1834 (1997)); and micropipette method (cf. "Kinematics of red cell aspiration by fluorescence-imaged microdeformation" by Discher D. E., Mohandas N. in Biophys J.71 (4)1680-1694(1996)).

Among these, the microchannel method is used commonly.

It is preferred that the present blood fluidity-improving agent be administered to persons whose whole blood transit time ranges from 10 to 1,000 seconds.

To measure effects achieved by the ingredients to
promote the blood circulation, a number of methods may be employed including: laser Doppler blood flowmeter method (cf. "Laser Doppler perfusion imaging of skin blood flow using red and near-infrared sources" by Abbot N.C., Ferrell W.R., Lockhart J.C. and Lowe J.G. in J Invest Dermatol 107 882-886 (1996)); transcutaneous measurement of oxygen partial pressure (cf. "Infrapopliteal interventions for limb salvage in diabetic patients" by Hanna G.P., Fujise K., Kjellgren O., Feld S. Fife C., Schroth G., Clanton T., Anderson V., Smalling R. in J.Am.Coll.Cardiol 30 664-669 (1997)); and cold water immersion test (cf. "Comparison of alpha-tocopheryl nicotinate and acetate on skin microcirculation" by Kamimura M. in Am.J.Clin.Nutr. 27 1110-1116 (1974)). Among these, the cool water immersion test is commonly used.

It is preferred that the chlorogenic acids be administered to
persons who have a 10 min. or longer skin temperature recovery time of their hands or fingertips as measured in a cold water immersion test, in which the hands or fingertips are immersed in cold water (at 15 °C for 5 minutes) and the time (elapsing for temperature recovery to 25 °C) after removal from the cold water is measured, in other words it is preferred persons requiring a 10 minutes or longer recovery time of finger surface temperature after cold water loading are administered.

According to the present invention, one or more ingredients selected from the group consisting of chlorogenic acids, and pharmaceutically acceptable salts of these acids may be used effectively as a blood-fluidity-improving agent, a blood circulation promoter, a body coldness-improving agent, or a body temperature decrease-inhibiting agent. The blood fluidity-improving agent and, blood circulation promoter may contain such active ingredients in a quantity ranging preferably from about 0.01 to about 80 wt%, more preferably from about 0.05 to about 60 wt% and particularly preferably from about 0.1 to about 60 wt%.

Compositions for oral use include, for example, tablets, granules, subtle granules, pills, powdered drugs, capsules (including hard capsules and soft capsules), lozenges, chewable tablets, food supplements, etc. available as solid or powdered preparations, as appropriate. These preparations cited just above may be provided as foods including supplements for nutritional or dietary purpose.

These preparations may preferably contain the active ingredients in a quantity ranging preferably from about 0.1 to about 80 wt% and particularly preferably from about 10 to about 60 wt% in view of effective uptake per day.

When using chlorogenic acids as foods, such foods
may be provided in any appropriate forms containing conventional food additives in addition to the present active ingredients, including, besides the aforementioned preparations: beverages, soy sauces, milk, yoghurts, soybean pastes and like liquid, emulsified or paste foods; jellies, gummis and like semisolid foods; and cookies, gums, soybean curds *(tofu),* etc.

These liquid, emulsified or paste foods, semisolid foods may preferably contain the active ingredients of the present invention in a quantity ranging preferably from about 0.01 to about 50 wt% and particularly preferably from about 0.05 to about 10 wt% in view of effective uptake per day.

The blood fluidity-improving agent, and blood circulation promoter of the present invention not only have an excellent safety to permit healthy persons to take in daily without causing any problems, but also may be used as food supplements in the form of tablets, granules, etc., various beverages, or various foods, especially foods for specified health use.

### Examples

### Applicable test 1 Evaluation of blood fluidity

### i) Experimental materials and methodology

### (a) Animals used

Tests were started using stroke-prone spontaneously hypertensive rats (SHRSP/Izm, male) 6 weeks old after acclimating the rats 1 week or longer. The rats were all fed under the conditions of 20 to 26 °C temperature, 40 to 70 % humidity and 12 hr. lighting time (from 6 a.m. to 6 p.m.).

### (b) Method of administration and applied doses

The rats were administered with samples of the example of the present invention and samples of the comparative example once a day for continuous 28 days from a day when the rats reached 8 weeks of age. For administration, samples were orally administered by forced administration using a disposable polypropylene syringe attached with a metallic oral sonde. For the example, each sample contained a chlorogenic acid (supplied by Sigma Chemical Co., Ltd.) of 50 mg/Kg (body weight)/day, while the comparative example used the equal dose of injection grade water (supplied by Otsuka Pharmaceutical Co., Ltd., Tokyo, Japan) as samples. The preferred example also used the injection grade water as a medium for samples.

For feeds, the rats had been fed freely with a solid feed (CRF-1 supplied by Oriental Yeast Co., Ltd., Tokyo, Japan) for a period from the arrival to grouping of rats and with another solid feed (SP (a feed containing 0.4 % common salt) supplied by the same manufacturer as above) for a period succeeding to the grouping. For drinking water, tap water was used in the period from the receipt to grouping of rats and 1 % brine in the period after the grouping, respectively, for free uptake.

### (c) Test method

After administering rats with a specified sample for 28 continuous days, blood was sampled from each rat using a VENOJECT II vacuum blood collection tube of 7 mL size (available from Terumo Corporation, Tokyo, Japan) preinfused with 350 µL heparin as an anticoagulant (equivalent to 5 % of whole blood). Upon sampling, the blood was agitated quickly to obtain blood samples for measurement. For blood measurement, a cellular microrheological measurement system was used (MC-FAN available from Hitachi Haramachi Electronics Co., Ltd., Ibaragi, Japan). The system incorporates an array of microchannels as a model of blood capillaries to measure the flowing characteristics of blood cells under constant differential pressure. As the model of blood capillaries, a silicon monocrystal basal plate having microchannels of 7 µm in width was used.

First, the time was measured for a 100 µL physiological saline solution to pass through the blood capillary model under differential pressure of 20 cm water column (the measured value to be used later for correction). Then the time was measured (in sec.) for a 100 µL each blood sample to pass through the blood capillary model under the same conditions. The time was measured every 10 µL fraction of sample flow. Each blood sample was microscopically observed as it flowed through the blood capillary model. Using the thus measured transit times as a measure of the blood fluidity, the blood samples were evaluated for their improvement in their fluidity based on the criteria that the shorter transit times represent higher levels of improvement.

### ii) Test results

As is clearly seen from table 1 below showing the test result, the preferred example (a group of rats fed with the chlorogenic acid) exhibit shorter transit times than the comparative example (a group of rats fed with injection grade water), indicating an improvement of the blood fluidity in the preferred example.

**Table 1**

| Blood volume passed | Transit time (sec.) | |
|---|---|---|
| µL | Preferred example | Comparative example |
| 10 | 4.87 | 5.46 |
| 20 | 9.67 | 10.59 |
| 30 | 14.31 | 15.64 |
| 40 | 19.34 | 20.73 |
| 50 | 24.59 | 26.10 |
| 60 | 30.03 | 32.24 |
| 70 | 35.90 | 37.93 |
| 80 | 41.65 | 43.70 |
| 90 | 47.58 | 49.90 |
| 100 | 53.38 | 56.34 |

### Applicable test 2 Evaluation of peripheral circulation function by cold water immersion test (cooling-rewarming test)

### i) Experimental materials and methodology

As the preferred example of the present invention, subjects of 5 healthy women having a depressed peripheral circulation function each were allowed to drink bottled 125 mL vegetable-fruit mixed juice beverage containing a green coffee beans extract (with 140 mg (0.1 wt%) content as chlorogenic acid) daily one bottle for 6 continuous weeks. As the comparative example, the same 5 subjects, after 3 weeks from the end of the above 6 weeks period of drinking for the preferred example, each were allowed to drink bottled 125 mL vegetable-fruit mixed juice beverage not containing the green coffee beans extract (with 6 mg (0.005 wt%) content as chlorogenic acid) daily one bottle for further 6 continuous weeks. The subjects were evaluated for their peripheral circulation functions through cold water immersion test. After acclimation at 20 °C (50 % RH) for 30 minutes, the 5 subjects were tested by immersing their left hands to wrist depth in 15 °C cold water for 5 minutes and the recovery of palmar skin surface temperature of the third digit distal phalanxes of their left hands was measured (using Anritsu HPD-2236 DIGITAL THERMOMETER available from Anritsu Corporation, Kanagawa, Japan).

### ii) Test results

The test result is shown in Table 2 below. The temperatures shown in Table 2 were measured 45 minutes after removal of the left hands from the cold water in the test before and after the subjects had drunk the samples of the preferred example and the comparative example, respectively. As is clearly seen from Table 2, the preferred example exhibits a significant increase in body temperature over the comparative example, indicating that the uptake of the chlorogenic acid has the effect of improving the peripheral circulation function, namely blood circulation. Besides, it was observed in the experiment that the improved blood circulation brought about certain improvements in body coldness and inhibition of body temperature decrease.

**Table 2 Temperature recovery in cold water immersion test (skin temperatures 45 min. after end of immersion)**

| | Before uptake | After 6 weeks uptake |
|---|---|---|
| Preferred example | 20.5±1.0 | 22.8±2.0 |
| Comparative example | 21.7±1.3 | 22.2±1.4 |

| | | |
|---|---|---|
| Mean ± standard error (n=5) * p<0.05 vs. before uptake (Wilcoxon signed rank test) | | |

### Preferred example 1

Here, is disclosed an example of the present ingredients used for a beverage.

| | |
|---|---|
| Skimmed milk | 3.5 (wt%) |
| Enzymatically decomposed milk casein | 3.5 |
| Fructose | 9.0 |
| Chlorogenic acid | 0.3 |
| Sodium ferulate | 1.0 |
| Citric acid | 0.1 |
| Ascorbic acid | 0.1 |
| Flavor | 0.1 |
| Water | 82.4 |

The beverage of this preferred example having the formulation shown above had high storage stability and a good flavor.

## Claims

1. Non-therapeutic use of one or more chlorogenic acids selected from 3-caffeoylquinic acid, 4-caffeoylquinic acid, 5-caffeoylquinic acid, 3,4-dicaffeoylquinic acid, 3,5-dicaffeoylquinic acid, 4,5-dicaffeoylquinic acid, 3-feruloylquinic acid, 4-feruloylquinic acid, 5-feruloylquinic acid and 3-feruloyl-4-caffeoylquinic acid and pharmaceutically acceptable salts thereof for promoting the blood circulation.

2. Non-therapeutic use as defined in claim 1, wherein the peripheral circulation of person is improved.

3. Non-therapeutic use as defined in claim 1, wherein the temperature of the body is increased.

4. Non-therapeutic use as defined in any one of claims 1 to 3, wherein the chlorogenic acids are used in the composition in an amount of 0.01 to 80% by weight.

5. Non-therapeutic use as defined in any one of the claims 1 to 4, wherein said acids and salts thereof are taken as a food, food supplement or beverage.

6. Non-therapeutic use as defined in any one of the claims, wherein basic substances to form the salts of the chlorogenic acids are alkali metal hydroxides, hydroxides of alkaline earth metals, ammoniumhydroxide, basic amino acids, or mono-, di- or triethanolamine.

## Patentansprüche

1. Nicht-therapeutische Verwendung von einer oder mehreren Chlorogensäuren, ausgewählt aus 3-Kaffeoylchinasäure, 4-Kaffeoylchinasäure, 5-Kaffeoylchinasäure, 3,4-Dikaffeoylchinasäure, 3,5-Dikaffeoylchinasäure, 4,5-Dikaffeoylchinasäure, 3-Feruloylchinasäure, 4-Feruloylchinasäure, 5-Feruloylchinasäure und 3-Feruloyl-4-kaffeoylchinasäure und pharmazeutisch akzeptablen Salzen davon zur Förderung der Blutzirkulation.

2. Nicht-therapeutische Verwendung wie in Anspruch 1 definiert, worin die periphere Zirkulation einer Person verbessert wird.

3. Nicht-therapeutische Verwendung wie in Anspruch 1 definiert, worin die Temperatur des Körpers erhöht wird.

4. Nicht-therapeutische Verwendung wie in einem der Ansprüche 1 bis 3 definiert, worin die Chlorogensäuren in der Zusammensetzung in einer Menge von 0,01 bis 80 Gew.-% verwendet werden.

5. Nicht-therapeutische Verwendung wie in einem der Ansprüche 1 bis 4 definiert, worin die Säuren und Salze davon als Nahrungsmittel, Nahrungsergänzungsmittel oder Getränk aufgenommen werden.

6. Nicht-therapeutische Verwendung wie in einem der Ansprüche definiert, worin basische Substanzen zur Bildung der Salze der Chlorogensäuren Alkalimetallhydroxide, Hydroxide von Erdalkalimetallen, Ammoniumhydroxid, basische Aminosäuren oder Mono-, Di- oder Triethanolamin sind.

## Revendications

1. Utilisation non thérapeutique d'un ou de plusieurs acides chlorogéniques sélectionnés parmi l'acide 3-caféoylquinique, l'acide 4-caféoylquinique, l'acide 5-caféoylquinique, l'acide 3,4-dicaféoylquinique, l'acide 3,5-dicaféoylquinique, l'acide 4,5-dicaféoylquinique, l'acide 3-féruloylquinique, l'acide 4-féruloylquinique, l'acide 5-féruloylquinique et l'acide 3-féruloyl-4-caféoylquinique et des sels pharmaceutiquement acceptables de ceux-ci pour favoriser la circulation sanguine.

2. Utilisation non thérapeutique selon la revendication 1, dans laquelle la circulation périphérique d'une personne est améliorée.

3. Utilisation non thérapeutique selon la revendication 1, dans laquelle la température du corps est augmentée.

4. Utilisation non thérapeutique selon l'une quelconque des revendications 1 à 3, dans laquelle les acides chlorogéniques sont utilisés dans la composition en une quantité de 0,01 à 80 % en poids.

5. Utilisation non thérapeutique selon l'une quelconque des revendications 1 à 4, dans laquelle lesdits acides et sels de ceux-ci sont pris comme aliment, complément alimentaire ou boisson.

6. Utilisation non thérapeutique selon l'une quelconque des revendications précédentes, dans laquelle des substances basiques pour former les sels des acides chlorogéniques sont des hydroxydes de métal alcalin, des hydroxydes de métaux terreux alcalins, de l'hydroxyde d'ammonium, des acides aminés basiques, ou de la mono-, di- ou triéthanolamine.
